(19) Europäisches Patentamt
European Patent Office
Office européen des brevets

(11) Numéro de publication: **0 066 484**
**B1**

# (12) FASCICULE DE BREVET EUROPEEN

(45) Date de publication du fascicule du brevet: **08.08.84**

(51) Int. Cl.[3]: **C 07 D 207/404,** C 07 D 209/48, C 07 D 205/10, C 07 D 211/88

(21) Numéro de dépôt: **82400805.6**

(22) Date de dépôt: **03.05.82**

(54) **Procédé de préparation d'imides N-benzylsubstitués.**

(30) Priorité: **15.05.81 FR 8109698**

(43) Date de publication de la demande: **08.12.82 Bulletin 82/49**

(45) Mention de la délivrance du brevet: **08.08.84 Bulletin 84/32**

(84) Etats contractants désignés: **AT BE CH DE FR GB IT LI LU NL SE**

(56) Documents cités:
**DE - A - 2 526 652**

**METHODEN DER ORGANISCHEN CHEMIE, Houben-Weyl, 1957, Tome XI/1, Georg Thieme Verlag, pages 795-805, Stuttgart (DE); "Stickstoffverbindungen II; Amine"**

**Le dossier contient des informations techniques présentées postérieurement au dépôt de la demande et ne figurant pas dans le présent fascicule.**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526 Chemin du Bois, F-69140 Rillieux (FR)**
Inventeur: **Reppelin, Michel, 10 Chemin Neuf, F-69660 Collonges-au-Mont d'Or (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 066 484 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de préparation d'imides N-benzylsubstitués.

Les imides N-benzylsubstitués visés par la présente invention ont pour formule générale: ·

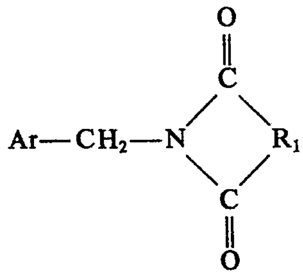

(I)

dans laquelle Ar représente un radical benzénique et $R_1$ représente un groupement hydrocarboné aliphatique ou aromatique.

On entend au sens de la présente invention par radical benzénique, le radical phényle ou un radical phényle substitué.

On connaît dans l'art antérieur Methoden der organischen Chemie, Houben Weyl, 1957, Teme XI/1, pages 795—805, qui décrit la condensation de dérivé benzénique avec le N-hydroxyméthylphtalimide pour donner des N-benzylphtalimides. La réaction est effectuée dans l'acide sulfurique.

Est aussi décrit dans la demande de brevet allemand N° 2 256 652 la condensation du trifluorméthylbenzène avec le N-hydroxyméthylphtalimide dans l'acide sulfurique à une température de 50° C pour donner le N(trifluorométhylbenzyl)phtalimide.

L'utilisation d'acide sulfurique présente des inconvénients certains au plan industriel. En effet, le produit de la réaction se dissout dans l'acide sulfurique; de plus, on est en présence d'eau formée lors de la réaction; la distillation de l'eau et de 1'$H_2SO_4$ est pratiquement impossible car elle requiert des niveaux thermiques incompatibles avec la stabilité des produits recherchés. Il faut donc soit dilner à l'eau pour pouvoir extraire avec un solvant organique, soit extraire en milieu acide sulfurique concentré ce qui est de mise en oeuvre compliquée et onéreuse. De plus, dans le cas de composés fluorés (comme dans le cas du brevet allemand précité) la présence de $H_2SO_4$ induit des réactions de défluoration du groupement fluoré ce qui est préjudiciable à la fois au plan économique et au plan technique.

Enfin, les propriétés oxydantes et sulfonantes de l'acide sulfurique provoquent la formation de mombreux produits secondaires.

On voit donc que substiste dans l'art antérieur le besoin d'un procédé de préparation d'imides N-benzylsubstitués permettant d'obtenir des rendements économiquement satisfaisants et de s'affranchir des difficultés dues à l'acide sulfurique.

La présente invention atteint cet objectif.

La présente invention a pour objet un procédé de préparation d'imides N-benzylsubstitués caractérisé en ce que l'on fait réagir en présence d'acide fluorhydrique un composé de formule:

ArH (II)

dans laquelle Ar représente un radical benzénique avec un composé de formule:

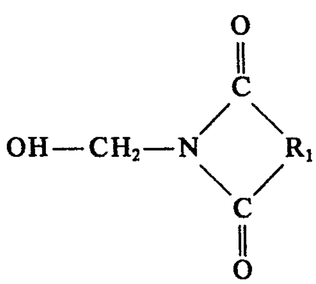

(III)

dans laquelle $R_1$ représente un groupement hydrocarboné aliphatique ou aromatique ayant de 1 à 12 atomes de carbone.

L'acide fluorhydrique mis en oeuvre dans le cadre du procédé selon l'invention est de préférence de l'acide fluorhydrique anhydre.

On peut également utiliser de l'acide fluorhydrique aqueux ayant une concentration de préférence supérieure à 90%.

Selon une mode de mise en oeuvre particulier du procédé selon l'invention, Ar représente dans la formule II le radical phényle.

Selon un autre mode de mise en oeuvre particulier du procédé selon l'invention, Ar représente dans

la formule II, le radical phényle substitué par au moins un radical choisi parmi le groupe comprenant F, Cl, Br, CN, $NO_2$, $NH_2$, CHO, COOH, COR, COOR ou R est un radical alkyle ayant de 1 à 6 atomes de carbone, les radicaux alkyle et alkoxy ayant de 1 à 6 atomes de carbone, le radical phényle, le radical phénoxy et les radicaux $CF_3$, $SCF_3$, $OCF_3$.

Le procédé selon l'invention est particulièrement bien adapté à la mise en oeuvre des composés de formule III dans laquelle $R_1$ est choisi parmi le groupe comprenant les radicaux

$—CH_2—CH_2—CH_2—$ $—CH_2—CH_2—$ 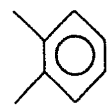

On utilise, de préférence, une quantité d'acide fluorhydrique telle que le rapport molaire de l'acide fluorhydrique au composé II est compris entre 5 et 50.

Les composés II et III sont utilisés de préférence en quantités telles que le rapport molaire de II à III est compris entre 0,5 et 2.

Pour une bonne mise en oeuvre du procédé selon l'invention, on opère à température comprise, de préférence, entre 0 et 100° C.

Lorsque la température sera supérieure à la température d'ébullition de 1'HF (20° C), on opérera dans un réacteur fermé sous pression autogène. Dans le cas contraire, on préférera opérer sous pression atmosphérique, bien que des pressiors supérieures ou inférieures à la pression atmosphérique ne soient pas exclues du domaine de l'invention.

Les temps de réaction sont généralement compris entre quelques minutes et 20 heures.

La séparation des produits de la réaction peut se faire par toute méthode connue de l'homme de l'art et, en particulier, par coulée du mélange réactionnel brut sur de la glace et extraction à l'aide de solvants organiques ou par distillation de 1'HF (distillation flash par exemple).

On peut citer comme exemples non limitatifs de composés de formule II, les composés suivants: trifluorométhylbenzène, fluorobenzène, trifluorométhoxybenzène, trifluorométhylthiobenzène, chlorobenzène, phénol, anisole, veratrol, gaïacol, toluène, nitrobenzène, oxyde de phényle, o-cresol, m-cresol, p-cresol, éthylbenzène, benzonitrile, aniline, acétophénone, acide benzoïque, les di et trichlorobenzènes, les chloronitrobenzènes, les chlorotoluènes, les nitrotoluènes, le biphényle, les chlorotrifluorométhylbenzènes, les bromotrifluorométhylbenzènes, les fluorotoluènes, les chlorotrifluorométhoxybenzènes, métabistrifluorométhylbenzène.

On peut citer comme exemples non limitatifs de composés de formule III, les composés suivants: N-hydroxyméthylsuccinimide, N-hydroxyméthylglutarimide, N-hydroxyméthylphtalimide, N-hydroxyméthyl, méthyl-2 succinimide, N-hydroxyméthyldiméthyl-2,3 succinimide, N-hydroxyméthyl phényl-2 succinimide, N-hydroxyméthyldiméthyl-2,4 glutarimide.

Les composés suivants sont des exemples de composés de formule I pouvant être préparés par le procédé de l'invention: N-métatrifluorométhylbenzylphtalimide, N-métabistrifluorométhylbenzylphtalimide, N-dichloro-2,4 benzylphtalimide, N-ortho et N-paratrifluorométhoxybenzylphtalimide, N-ortho et N-para fluorobenzylphtalimide, N-ortho et N-paratrifluorométhylthiobenzylphtalimide.

On peut également citer les dérivés correspondant du succinimide et du glutarimide.

Les composés obtenus sont intéressants notamment pour la synthése d'intermédiaires pour la préparation de composés ayant une activité pharmaceutique ou phytosanitaire.

Ils peuvent donner naissance par traitement à l'hydrazine ou ou hydrolyse aux amines $Ar—CH_2—NH_2$ (voir brevet allemand 2 526 652).

Les exemples qui vont suivre illustrent plus complètement l'invention. Ils ne sauraient en constituer une limitation quelconque.

## Exemple I:

Préparation du N-métatrifluorométhylbenzylphtalimide

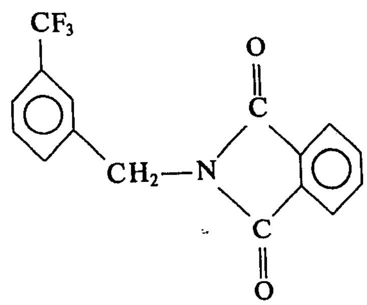

Dans un réacteur en acier inoxydable, on introduit 90 g (4,5 moles) d'HF anhydre que l'on refroidit à 0° C environ. On introduit ensuite lentement 17,7 g (0,1 mole) d'hydroxyméthylphtalimide en maintenant la température entre 0 et 5° C. On introduit ensuite goutte à goutte 17 g (0,116 mole) de trifluorométhylbenzène. On ferme le réacteur et on le porte sous agitation à 50° C pendant 20 heures. On refroidit alors à 0—5° C.

On verse le mélange réactionnel sur 200 g de glace et on extrait la phase organique au chlorure de méthylène (5 · 100 $cm^3$). La phase organique est ensuite lavée à l'eau déminéralisée jusqu'à obtention d'une eau de lavage ayant un pH compris entre 5 et 6. On sèche et on évapore sous pression réduite (15—20 mm de Hg). On récupère 30 g d'un produit dont l'analyse par chromatographie en phase vapeur, infrarouge et spectrométrie de masse révèle qu'il s'agit du N-métatrifluorométhylbenzylphtalimide.

Exemple 2:

Préparation du N-métatrifluorométhylbenzylsuccinimide

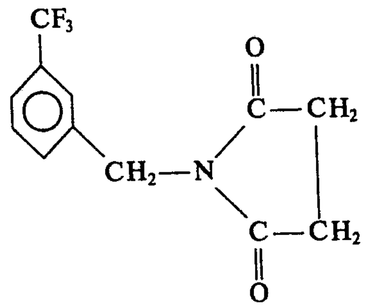

En opérant comme dans l'exemple 1, on met en oeuvre 100 g d'HF anhydre (5 moles) 29,2 g de trifluorométhylbenzène (0,2 mole), 30 g (0,23 mole) d'hydroxymethylsuccinimide.

On porte à 50° C pendant 22 heures.

On obtient 46,8 g du composé attendu.

Exemple 3:

Préparation des N-ortho et N-parafluorobenzylsuccinimides

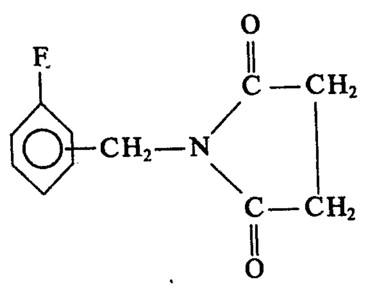

On opère comme dans l'exemple 1 en utilisant 100 g d'HF anhydre (5 moles), 19,2 g (0,2 mole) de fluorobenzène et 30 g (0,23 mole) d'hydroxyméthylsuccinimide.

La réaction est conduite à 50° C pendant 4 heures.

On obtient 40 g d'un mélange contenant 20% d'orthofluorobenzylsuccinimide et 30% de parafluorobenzylsuccinimide.

Exemple 4:

Préparation des N-ortho et N-paratrifluorométhylthiobenzylsuccinimide

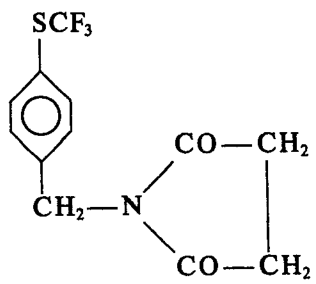

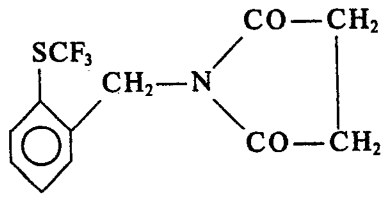

On opère comme dans l'exemple 1 en utilisant 100 g d'HF anhydre (5 moles), 17,8 g (0,1 mole) de trifluorométhylthiobenzène et 12,9 g (0,1 mole) de N-hydroxyméthylsuccinimide.

La réaction est conduite à 20° C pendant 3 heures 30 minutes.

On obtient 23,8 g d'un mélange contenant environ 20% de N-orthotrifluorométhylthiobenzylsuccinimide et environ 80% de N-paratrifluorométhylthiobenzylsuccinimide.

Exemple 5:

Préparation des N-ortho et N-paratrifluorométhoxybenzylsuccinimide

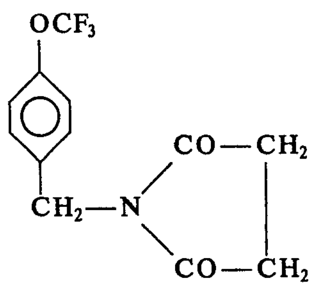

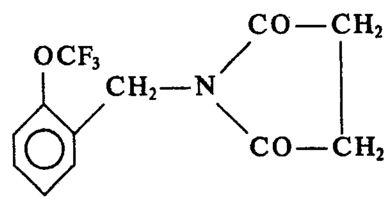

On opère comme dans l'exemple 1 en utilisant 100 g d'HF anhydre (5 moles), 16,2 g (0,1 mole) de trifluorométhoxybenzène et 12,9 g (0,1 mole) de N-hydroxyméthylsuccinimide.

La réaction est conduite à 10° C pendant 3 heures.

On obtient 18 g d'un mélange contenant eviron 15% de N-orthotrifluorométhoxybenzylsuccinimide et environ 85% de N-paratrifluorométhoxybenzylsuccinimide.

Exemple 6:

Préparation du N-paratrifluorométhylthiobenzylphtalimide

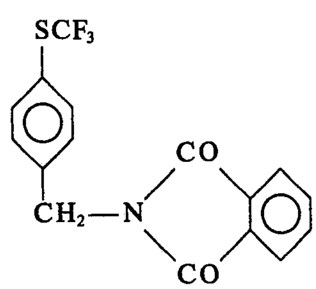

On opère comme dans l'exemple 1 en utilisant 50 g d'HF anhydre (2,5 moles), 9 g (0,05 moles) de trifluorométhylthiobenzène et 9 g (0,05 moles) de N-hydroxyméthylphtalimide.

La réaction est conduite à 20° C pendant 4 heures.

On obtient 14,7 g de N-paratrifluorométhylthiobenzylphtalimide.

Exemple 7:

Préparation des N-ortho et N-parabromobenzylglutarimide

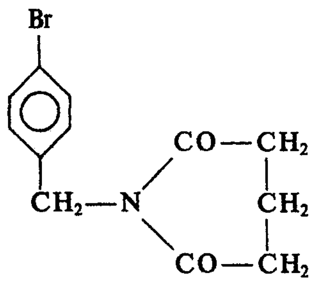

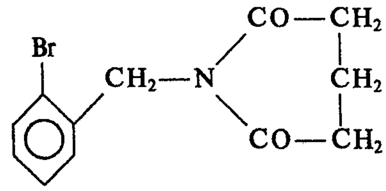

On opère comme dans l'exemple 1 en utilisant 100 g d'HF anhydre (5 moles), 31,4 g (0,2 mole) de bromobenzène et 35,7 g (0,25 mole) de N-hydroxyméthylglutarimide.

La réaction est conduite à 25° C pendant 5 heures.

On obtient 47,3 g d'un mélange de N-orthobromobenzylglutarimide et de N-parabromobenzylglutarimide.

**Revendications**

1. Procédé de préparation d'imides N-benzyl caractérisé en ce que l'on fait réagir, en présence d'acide fluorhydrique, un composé de formule:

ArH (II)

dans laquelle Ar représenté un radical benzénique avec un composé de formule:

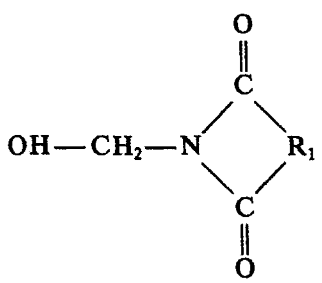

(III)

dans laquelle $R_1$ représente un groupement hydrocarboné aliphatique ou aromatique ayant de 1 à 12 atomes de carbone.

2. Procédé selon la revendication 1 caractérisé en ce que l'acide fluorhydrique mis en oeuvre est de l'acide fluorhydrique anhydre.

3. Procédé selon l'une quelconque des revendications précédentes caractérisé en ce que dans la formule II, Ar représente le radical phényle.

4. Procédé selon l'une quelconque des revendications précédentes caractérisé en ce que dans la formule II, Ar représente un radical phényle substitué par au moins un radical choisi parmi le groupe comprenant F, Cl, Br, CN, $NO_2$, $NH_2$, CHO, COOH, COR, COOR où R est un radical alkyle ayant de 1 à 6 atomes de carbone, les radicaux alkyle et alkoxy ayant de 1 à 6 atomes de carbone, le radical phényle et phénoxy, $CF_3$, $SCF_3$, $OCF_3$.

5. Procédé selon l'une quelconque des revendications précédentes caractérisé en ce que dans la formule III, $R_1$ est choisi parmi le groupe comprenant $-CH_2-CH_2-$, $-CH_2-CH_2-CH_2-$ et phénylène.

6. Procédé selon l'une quelconque des revendications précédentes caractérisé en ce que l'on utilise une quantité d'acide fluorhydrique telle que le rapport molaire de l'acide fluorhydrique au composé II est compris entre 5 et 50.

7. Procédé selon l'une quelconque des revendications précédentes caractérisé en ce que l'on utilise les composés II et III en quantités telles que le rapport molaire du composé II au composé III est compris entre 0,5 et 2.

8. Procédé selon l'une quelconque des revendications précédentes caractérisé en ce que l'on opère à une température comprise entre 0° C et 100° C.

**Patentansprüche**

1. Verfahren zur Herstellung von N-Benzylimiden, dadurch gekennzeichnet, daß man eine Verbindung der Formel:

ArH (II)

in der Ar ein Benzolrest ist, mit einer Verbindung der Formel:

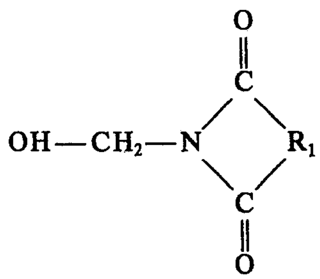

(III)

in der $R_1$ eine aliphatische oder aromatische Kohlenwassergruppe mit 1 bis 12 C-Atome bedeutet, in Anwesenheit von Fluorwasserstoffsäure umsetzt.

2. Verfahren nach Anspruch 1, dadurch gekennzeichnet, daß die eingesetzte Fluorwasserstoffsäure wasserfrei ist.

3. Verfahren nach einem der vorangehenden Ansprüche, dadurch gekennzeichnet, daß Ar in der Formel II den Phenylrest bedeutet.

4. Verfahren nach einem der vorangehenden Ansprüche, dadurch gekennzeichnet, daß Ar in der Formel II einen Phenylrest bedeutet, der durch mindestens einen Rest substituiert ist, der aus der Gruppe von F, Cl, Br, CN, $NO_2$, $NH_2$, CHO, COOH, COR, COOR gewählt wird, wobei R ein Alkylrest mit 1 bis 6 Kohlenstoffatomen ist, Alkyl- und Alkoxyreste 1 bis 6 Kohlenstoffatomen, den Phenyl- und Phenoxyrest $CF_3$, $SCF_3$ oder $OCF_3$ ist.

5. Verfahren nach einem der vorangehenden Ansprüche, dadurch gekennzeichnet, daß $R_1$ in der Formel III aus der Gruppe von $-CH_2-CH_2-$, $-CH_2-CH_2-CH_2-$ und Phenylen gewählt wird.

6. Verfahren nach einem der vorangehenden Ansprüche, dadurch gekennzeichnet, daß man eine solche Menge an Fluorwasserstoffsäure verwendet, daß das Molverhältnis der Fluorwasserstoffsäure zur Verbindung II zwischen 5 und 50 liegt.

7. Verfahren nach einem der vorangehenden Ansprüche, dadurch gekennzeichnet, daß man solche Menge der Verbindungen II und III verwendet, daß das Molverhältnis der Verbindung II zur Verbindung III zwischen 0,5 und 2 liegt.

8. Verfahren nach einem der vorangehenden Ansprüche, dadurch gekennzeichnet, daß man bei einer Temperatur zwischen 0° C und 100° C arbeitet.

**Claims**

1. Process for the preparation of N-benzyl-imides characterised in that a compound of the formula:

ArH (II)

in which Ar represents a benzene radical is reacted with a compound of the formula:

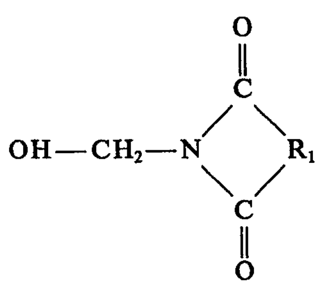

(III)

in which $R_1$ represents an aliphatic or aromatic hydrocarbon group having from 1 to 12 carbon atoms, in the presence of hydrofluoric acid.

2. Process according to claim 1, characterised in that the hydrofluoric acid employed is anhydrous

hydrofluoric acid.

3. Process according to any one of the preceding claims, characterised in that in formula II Ar represents the phenyl radical.

4. Process according to any one of the preceding claims, characterised in that in formula II Ar represents a phenyl radical substituted by at least one radical chosen from amongst the group comprising F, Cl, Br, CN, $NO_2$, CHO, COOH, COR and COOR (where is an alkyl radical having from 1 to 6 carbon atoms), alkyl and alkoxy radicals having from 1 to 6 carbon atoms, the phenyl and phenoxy radical, $CF_3$, $SCF_3$ and $OCF_3$.

5. Process according to any one of the preceding claims, characterised in that in formula III $R_1$ is chosen from the group comprising $-CH_2-CH_2-$, $-CH_2-CH_2-CH_2$ and phenylene.

6. Process according to any one of the preceding claims, characterised in that the amount of hydrofluoric acid used is such that the molar ratio of hydrofluoric acid to compound II is between 5 and 50.

7. Process according to any one of the preceding claims, characterised in that the compounds II and III are used in such amounts that the molar ratio of compound II to compound III is between 0.5 and 2.

8. Process according to any one of the preceding claims, characterised in that it is carried out at a temperature of between 0° C and 100° C.